# EUROPEAN PATENT SPECIFICATION

(11) **EP 3 510 972 B1**
(45) Date of publication and mention of the grant of the patent: **26.07.2023**
(21) Application number: 17847968.9
(22) Date of filing: 28.06.2017
(51) Int. Cl.: A61F 2/07, A61F 2/82, A61F 2/06

(54) **BRANCH LUMEN STENT AND LUMEN STENT SYSTEM**
ZWEIGLUMEN-STENT UND LUMINALES STENTSYSTEM
ENDOPROTHÈSE LUMINALE SECONDAIRE ET SYSTÈME D'ENDOPROTHÈSE LUMINALE

(30) Priority: 07.09.2016 CN 201610812180
(43) Date of publication of application: 17.07.2019
(73) Proprietor: Lifetech Scientific (Shenzhen) Co., Ltd., Shenzhen, Guangdong 518057 (CN)
(72) Inventor: XIAO, Benhao, Shenzhen Guangdong 518057 (CN); ZHANG, Deyuan, Shenzhen Guangdong 518057 (CN); SHU, Chang, Shenzhen Guangdong 518057 (CN); YIN, Chao, Shenzhen Guangdong 518057 (CN); WANG, Yifei, Shenzhen Guangdong 518057 (CN)
(74) Representative: Prinz & Partner mbB
(86) International application number: PCT/CN2017/090592
(87) International publication number: WO 2018/045795

(56) References cited:
- WO-A2-03/063729
- CN-A- 1 555 769
- CN-A- 105 496 603
- CN-U- 205 458 864
- CN-U- 205 494 093
- CN-Y- 2 845 771
- US-A1- 2003 070 676
- US-A1- 2003 236 567
- US-A1- 2005 154 447

## Description

This invention relates to the field of implantable medical devices, and more particularly relates to a branch lumen stent and a lumen stent system.

A lumen stent may be used to isolate an artery dissection or an arterial aneurysm in a vessel. If there is a branch vessel in a lesion region, at least two lumen stents are generally used in combination to prevent a main body lumen stent from blocking the blood supply of the branch vessel.

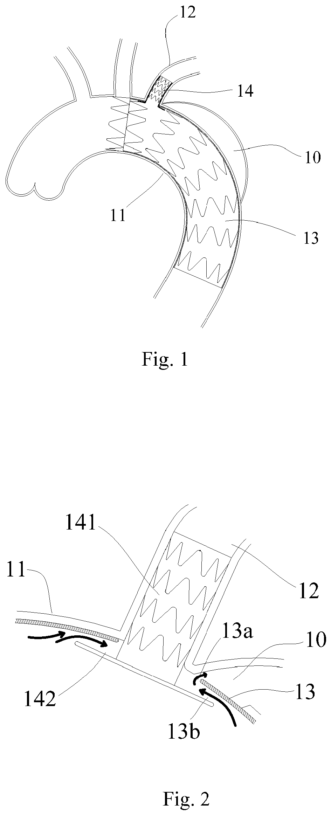

For example, referring to Figs. 1 and 2, an artery dissection 10 is located in an aorta arch 11 and extends to a location adjacent to a left subclavian artery 12. A main body lumen stent 13 may first be implanted into the aorta arch 11, and then a branch lumen stent 14 is implanted into the left subclavian artery 12 through a side hole of the main body lumen stent 13. The branch lumen stent 14 is also called a top hat stent because it is shaped like a top hat. The branch lumen stent 14 includes a tube body 141 and a border 142 surrounding an end opening of the tube body. The border 142 is basically perpendicular to the tube body 141 for abutting against the inner side wall of the main body lumen stent 13 to establish the blood supply between the aorta arch 11 and the left subclavian artery 12, and is clamped to prevent the branch lumen stent from falling off in the left subclavian artery 12, and to prevent the main body lumen stent 13 from moving under the impact of blood flow.

However, regardless of whether the side hole is formed on the side wall of the main body lumen stent 13 in vitro or in vivo to assemble the branch lumen stent 14, it is inevitable that the side hole and the branch lumen will not be completely concentric, thus the side hole may not be completely filled by the tube wall after the branch lumen stent 14 is implanted, and a gap 13a appears. Further, when a main body lumen has an irregular shape, the border 142 of the branch lumen stent 14 will not be completely adhered to the inner wall of the main body lumen stent 13. Moreover, continuous impact of the blood flow also may lead to the failure of the close connection of the branch lumen stent 14 and a connecting port of the main body lumen stent 13, thereby forming a gap 13b between the border 142 of the branch lumen stent 14 and the inner side wall of the main body lumen stent 13. The blood flow may enter a false lumen of the artery dissection 10 from the gap 13b through the gap 13a, thus forming a blood flow leakage channel as shown by an arrow in Fig. 2, thereby causing type-III endoleak.

This type-III endoleak may occur in the thoracic aorta, the abdominal aorta or other lumens. Continuous inflow of the blood flow may cause continuous enlargement of the false lumen of the dissection or aneurysm cavity, and finally lead to serious consequence such as rupture of the false lumen or the aneurysm cavity. Therefore, it is particularly important to avoid the type-III endoleak.

CN 205 494 093 U discloses a lumen stent comprising a tube body and a skirt arranged on and covering some of the tube body. The skirt comprises a flexible connecting section and a stent graft, wherein the flexible connection section is sealed and connected to the outer surface of the tube body. The proximal end of the stent graft is provided with a radial support structure, so that the stent graft has a radial self-expanding capability.

CN201949182U discloses another branch lumen stent.

### Summary of the Invention

According to the present invention a branch lumen stent suitable for implantation into a main body lumen stent is provided as defined in claim 1. The stent is capable of preventing type-III endoleak, and includes a tube body and a skirt arranged on the tube body with the skirt surrounding the tube body. The skirt includes a flexible connecting section and a stent graft. The distal end of the flexible connecting section is sealed and connected with the outer surface of the tube body, and the proximal end of the flexible connecting section is connected with the distal end of the stent graft. The proximal end of the stent graft is provided with a first radial support structure. When the flexible connecting section is radially compressed, at least a portion of the first radial support structure bends away from the tube body, so as to become approximately perpendicular to the axial direction of the tube body in the implanted condition.

An included angle between the flexible connecting section and the axial direction of the outer surface of the tube body is preferably 5 to 80 degrees. The maximum length of the first radial support structure is preferably less than or equal to the maximum perpendicular distance from the first radial support structure to the outer surface of the tube body. For example, the maximum perpendicular distance from the first radial support structure to the outer surface of the tube body is 6 to 40 mm, and the maximum length of the first radial support structure is 2 to 38 mm.

It is understood that the flexible connecting section has a connecting boundary connected with the tube body. The axial length of the flexible connecting section is preferably required to be less than the length from the proximal end surface of the tube body to the connecting boundary. For example, the value of the difference between the length from the proximal end surface of the tube body to the connecting boundary and the axial length of the flexible connecting section is not more than 20 mm.

The stent graft may be a straight tube shape or a horn shape. Also, the circumferential surface of the stent graft is a concave curved surface along a direction from the connecting boundary towards the proximal end, namely the diameter is decreased progressively and then increased progressively.

The first radial support structure may be all covered by a coating membrane, or only a portion of the first radial support structure is covered by the coating membrane, namely a portion of the first radial support structure is exposed from the proximal end.

The flexible connecting section may only include the coating membrane. One end of the coating membrane is sealed and connected with the outer surface of the tube body, and the other end of the coating membrane is sealed and connected with the stent graft.

The present invention also provides a lumen stent system as defined in claim 9.

According to the lumen stent and the lumen stent system provided by the present invention, the first radial support structure bends away from the tube body during compression of the flexible connecting section to become approximately perpendicular to the axial direction of the tube body when in the implanted condition. As a result the adhesion performance of the first radial support structure to the inner wall of the main body lumen stent may be effectively improved, so as to prevent the occurrence of the type-III endoleak.

### Brief Description of the Drawings

This disclosure will be further described below in conjunction with embodiments shown in the accompanying drawings. In the drawings:
Fig. 1 is a structure schematic diagram of a lumen stent system of the prior art;
Fig. 2 is a partially enlarged view of Fig. 1;
Fig. 3 is a schematic diagram of a main body lumen stent according to a first embodiment of the present invention;
Fig. 4 is a schematic diagram of a branch lumen stent according to the first embodiment of the present invention;
Fig. 5 is a schematic diagram of an axial section of the branch lumen stent in Fig. 4;
Figs. 6 to 10 are schematic diagrams showing the gradual release of the branch lumen stent in Fig. 4 from a delivery sheath;
Fig. 11 is a schematic diagram of an axial section of a branch lumen stent according to a comparative example, not covered by the invention;
Fig. 12A is a schematic diagram of a partial axial section of a branch lumen stent according to another comparative example, not covered by the invention;
Fig. 12B is a schematic diagram of the branch lumen stent in Fig. 12A after implantation;
Fig. 13 is a schematic diagram of a partial axial section of a branch lumen stent according to another comparative example, not covered by the invention;
Fig. 14 is a schematic diagram of a skirt of a branch lumen stent according to another comparative example, not covered by the invention;
Fig. 15A is a schematic diagram of a skirt of a branch lumen stent according to another comparative example, not covered by the invention;
Fig. 15B is a partially enlarged view of Fig. 15A;
Fig. 16A is a schematic diagram of first and second radial support structures of the branch lumen stent according to another comparative example, not covered by the invention; and
Fig. 16B is a partially enlarged view of Fig. 16A.

### Detailed Description of Embodiments

For a better understanding of the present invention, embodiments will be described in detail in conjunction with the accompanying drawings. To facilitate the description, a lumen is described by using a vessel as an example. The vessel may be an aortic arch, or a thoracic aorta, or an abdominal aorta and the like. The present disclosure is applicable to various other human lumens, such as a digestive tract lumen. In addition, in the description of the vessel, a direction may be defined according to a blood flow direction. In the present disclosure, it is defined that blood flow flows from the proximal end to the distal end. Unless otherwise specified, radial support structures of the present disclosure refer to closed wave-shaped annuluses that are axially disposed along the stent graft as is conventional in the art.

### First embodiment

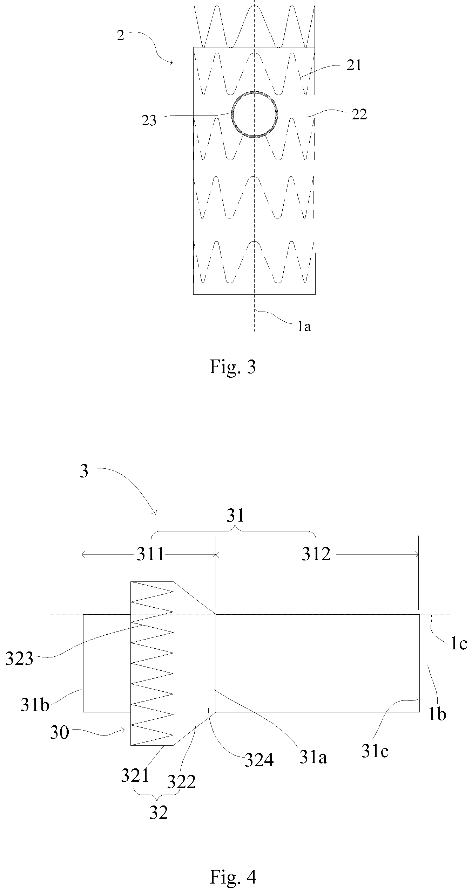

Referring to Fig. 3 and Fig. 4, a lumen stent system according to the first embodiment of the present invention includes a main body lumen stent 2 and at least one branch lumen stent 3 used cooperatively with the main body lumen stent 2.

The main body lumen stent 2 includes a tubular structure having an axial direction 1a. The tubular structure may be used as a new fluid channel after the main body lumen stent 2 is implanted into a lumen. For example, the tubular structure may be used as a new blood flow channel after the main body lumen stent 2 is implanted into a vessel. The main body lumen stent 2 includes a radial support structure 21 and a coating membrane 22 covering the radial support structure 21. The radial support structure 21 cooperates with the coating membrane 22 to form a side wall of the main body lumen stent 2. At least one side hole 23 is formed in the side wall, and is adapted to be matched in shape and size with the branch lumen stent 3, so that the branch lumen stent 3 may be combined with the main body lumen stent 2 through the side hole 23 and then implanted into a branch lumen. A radiopaque structure may be arranged at the periphery of the side hole 23. For example, one coil of radiopaque metal wire may be adhered to the edge of the side hole 23.

The radial support structure 21 may be made of various biocompatible materials including known materials used in manufacturing of an implantable medical device or a combination of various materials, such as 316L stainless steel, a cobalt-chromium-nickel-molybdenum-iron alloy, other cobalt alloys such as L605, tantalum, a nickel-titanium alloy (nitinol) or other biocompatible metals. The radial support structure 21 may be formed by winding a metal wire or cutting a metal tube, and may include a plurality of wave-shaped annuluses along the axial direction, such as multiple turns of Z-shaped waves, or include a helically wound structure, or include a mesh structure. The coating membrane 22 may be a PET (polyethylene terephthalate) membrane or a PTFE (polytetrafluoroethylene) membrane, which covers the radial support structure 21 by suturing or hot melting.

Through the radial support structure 21, the main body lumen stent 2 has a radial expandability, may be compressed under an external force, and restores to an initial shape through self-expansion or mechanical expansion (such as balloon dilatation expansion) and maintains the initial shape after the external force is withdrawn, so that after being implanted into the lumen, the main body lumen stent 2 can be secured within the lumen by its radial support against the lumen wall. It should be noted that unless otherwise specified in the following description, the initial shape of the lumen stent after radial deployment is described. Through the coating membrane 22, the main body lumen stent 2 may isolate a lesion region of the lumen. For example, the main body lumen stent 2 may isolate an artery dissection or an arterial aneurysm after being implanted into an artery vessel.

Referring to Fig. 4, the branch lumen stent 3 includes a tube body 31 and a skirt 32 arranged outside the tube body 31 in a manner where the skirt 32 surrounds the tube body 31. The tube body 31 includes a tubular structure having an axial direction 1b. The tubular structure may be used as a new fluid channel after the branch lumen stent 3 is implanted into a lumen. For example, the tubular structure may be used as a new blood flow channel after the branch lumen stent 3 is implanted into a vessel. The tube body 31 includes a radial support structure (not shown in the figure) arranged on the tube body and a coating membrane covering the radial support structure. The radial support structure cooperates with the coating membrane to form a side wall of the tube body 31. The same or similar radial support structure and coating membrane for the above-described main body lumen support 2 can be used, and will not be described herein. Through the radial support structure, the tube body 31 has a radial expandability, may be compressed under an external force, and restores to an initial shape through self-expansion or mechanical expansion (such as balloon dilatation expansion) and maintains the initial shape after the external force is withdrawn, so that after being implanted into a main body lumen, the tube body 31 can be secured within the lumen by its radial support against the lumen wall. Through the coating membrane, the tube body 31 may isolate a lesion region of the lumen. For example, the tube body 31 may isolate an artery dissection or an arterial aneurysm after the branch lumen stent 3 is implanted into an artery vessel.

The tube body 31 is divided into a first section 311 and a second section 312 along the axial direction, with the connecting boundary 31a of the tubular body 31 and the skirt 32 defining a boundary. The first section 311 is located on one side of the proximal end of the second section 312, namely the first section 311 extends from the connecting boundary 31a to a proximal-end opening end 31b of the tube body 31, and the second section 312 extends from the connecting boundary 31a to a distal-end opening end 31c of the tube body 31. It should be noted that the first section 311 and the second section 312 are only distinguished for facilitating the description, but does not represent that the tube body 31 is separated and disconnected at the above-mentioned connecting boundary 31a. The tube body 31 may be of a uniform integrated structure.

The skirt 32 includes stent graft 321 and a flexible connecting section 322 along the axial direction. The stent graft 321 is located on one side of the proximal end of the flexible connecting section 322. The stent graft 321 is substantially cylindrical and includes a first radial support structure 323 having a plurality of Z-shaped waves arranged along its circumferential direction. The wave heights of the plurality of Z-shaped waves may be equal or unequal. The stent graft 321 is suspended to form an opening 30. When a radial compression force is applied to the flexible connecting section 322, the flexible connecting section 322 would be radially compressed, and the opening end of the membrane-coated stent graft 321 will bend relative to the flexible connecting section 322 towards a direction away from the axial direction 1b. The skirt 32 includes a coating membrane 324. The coating membrane 324 seals and connects the connecting section 322 to the outer surface of the tube body 31.

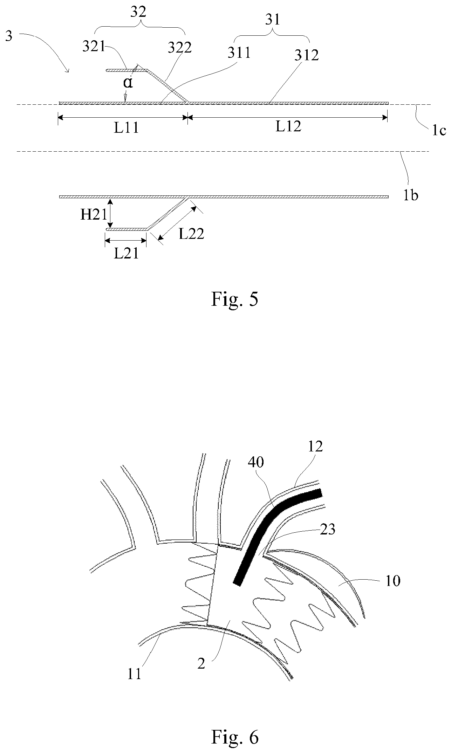

Still referring to Figs. 4 and 5, the flexible connecting section 322 includes the coating membrane 324 adjacent to the connecting boundary between the tube body 31 and the skirt 32. The coating membrane 324 may be a PET membrane or a PTFE membrane, which can seal and connect the flexible connecting section 322 to the outer surface of the side wall of the tube body 31 by suturing or hot melting. For example, the coating membrane 324 of the flexible connecting section 322 may be hot-melted together with the outer surface of the tube body 31 to achieve a sealed connection. Those ordinarily skilled in the art can select a proper sealing method as required, so that no more details will be described here. The coating membrane 324 may cover part of, or the whole of, the first radial support structure 323, or the first radial support structure 323 may be located in the middle region of the coating membrane 324. In the present embodiment, the flexible connecting section 322 is composed of the coating membrane 324 which may cover the first radial support structure 323 by hot melting or suturing. The coating membrane 324 is made of a flexible material, so that the stent graft 321 and the flexible connecting section 322 may be connected together in a bendable manner through the coating membrane 324.

The closed end of the flexible connecting section 322 is sealed and coupled to the tube body 31, and the other end of the flexible connecting section 322 radiates outwardly in the direction of the distal end towards the proximal end to form an approximately conical structure, namely the diameter of the flexible connecting section 322 increases progressively from the connecting boundary 31a to its opening end. An included angle α between the flexible connecting section 322 and the axial direction 1c of the outer surface of the tube body 31 is 5 to 80 degrees, or 5 to 60 degrees. The axial direction 1c of the outer surface of the tube body 31 is an axial direction along the contour of the outer surface. In the present embodiment, the tube body 31 is a straight tube, so that the axial direction 1c of the outer surface is parallel to the axial direction 1b of the tubular structure. If the tube body 31 is a conical tube, the axial direction 1c of the outer surface and the axial direction 1b generally form an acute included angle.

The length of the flexible connecting section 322 is less than that of the first section 311 of the tube body 31, and is equal to a length from the connecting boundary 31a to the bendable connecting boundary between the stent graft 321 and the flexible connecting section 322 along the axial direction of the outer surface of the flexible connecting section 322, and the length of the first section 311 is equal to a length from the connecting boundary 31a to the proximal-end opening 31b of the tube body 31 along the axial direction of the outer surface of the tube body 31. The value of the difference between the length of the flexible connecting section 322 and the length of the first section 311 of the tube body 31 is not more than 20 mm, for example, the difference value is 5 to 10 mm.

The first radial support structure 323 may be distributed on a portion of the stent graft 321, namely the maximum length of the first radial support structure 323 is smaller than the length of the stent graft 321 along the axial direction. The first radial support structure 323 also may be distributed over the whole stent graft 321, namely the maximum length of the first radial support structure 323 is equal to the length of the stent graft 321 along the axial direction. In the present embodiment, the first radial support structure 323 is distributed over the whole stent graft 321, and the proximal end of the first radial support structure is flush with the proximal end of the stent graft. The first radial support structure 323 has a radial expandability, may be compressed under an external force, and restores to an initial shape through self-expansion and maintains its initial shape after the external force is withdrawn. The first radial support structure 323 may be made of various biocompatible materials including known materials used in manufacturing of the implantable medical device or a combination of various materials, such as 316L stainless steel, cobalt-chromium-nickel-molybdenum-iron alloy, other cobalt alloys such as L605, tantalum, nickel-titanium alloy (nitinol) or other biocompatible metals. The first radial support structure 323 may include a plurality of wave-shaped annuluses along the axial direction, such as Z-shaped waves, or include a helically wound structure, or include a mesh structure.

The first radial support structure 323 may be formed by winding a metal wire having a diameter of 0.15 to 0.4 mm, or may be formed by cutting a metal tube. A wire diameter of a cut metal rod forming the first radial support structure 323 is 0.15 to 0.4 mm. In the present embodiment, the first radial support structure 323 is formed by winding a nickel-titanium alloy, and the diameter of the metal wire is 0.2 mm.

Along the direction of the opening 30 of the skirt 32, namely along the direction from the distal end to the proximal end, an included angle between the first radial support structure 323 and the axial direction 1b of the tube body 31 is more than or equal to 0 degree and less than 180 degrees, namely the orientation of the first radial support structure 323 is basically parallel to the axial direction 1b of the tube body 31, or turns outwardly relative to the axial direction 1b of the tube body 31; for example, turns perpendicularly outwardly relative to the axial direction 1b of the tube body 31.

The stent graft 321 has the first radial support structure 323 having the above orientation, which favorably enables the flexible connecting section 322 to actuate the opening end of the stent graft 321 to automatically bend outwardly relative to the flexible connecting section 322 in a radially compressed state, so as to form an approximately perpendicular border relative to the axial direction 1b of the tube body 31 after the first radial support structure 323 bends. In other words, after implantation, the flexible connecting section 322 actuates the stent graft 321 to bend under the radial compression of the branch lumen, so that the first radial support structure 323 is approximately perpendicular relative to the axial direction 1b of the tube body 31 so as to be adhered to the inner tube wall of the main body lumen stent 2. If the included angle between the first radial support structure 323 and the axial direction 1b of the tube body 31 is more than or equal to 0 degree and less than 90 degrees, the stent graft 321 (namely the first radial support structure 323) relatively turns outwardly (bends along a clockwise direction in the axial section in Figs. 4 and 5) under the radial compression of the flexible connecting section 322, so that the first radial support structure 323 may be approximately perpendicular relative to the axial direction 1b of the tube body 31. If the included angle between the first radial support structure 323 and the axial direction 1b of the tube body 31 is more than 90 degrees and less than 180 degrees, the stent graft 321 (namely the first radial support structure 323) relatively turns inwardly (bends along an anticlockwise direction in the axial section in Figs. 4 and 5) under the radial compression of the flexible connecting section 322, so that the first radial support structure 323 may be approximately perpendicular relative to the axial direction 1b of the tube body 31. If the included angle between the first radial support structure 323 and the axial direction 1b of the tube body 31 is approximately equal to 90 degrees, the first radial support structure 323 may be approximately perpendicular relative to the axial direction 1b of the tube body 31 in an initial state under the radial compression of the flexible connecting section 322.

The maximum length of the first radial support structure 323 is less than or equal to the maximum perpendicular distance from the first radial support structure 323 to the outer surface of the tube body 31. It is understood that when the wave heights of waveform units included in the first radial support structure 323 are unequal, the maximum length is the corresponding maximum wave height in all the waveform units. When the first radial support structure 323 includes a plurality of waveform units having equal wave heights, its maximum length is equal to a length from the distal end portion of the first radial support structure 323 to the proximal end portion of the first radial support structure 323 along the axial direction of the outer surface of the first radial support structure 323. The maximum length is 2 to 40 mm, for example 2 to 30 mm. The perpendicular distance from the first radial support structure 323 to the outer surface of the tube body 31 is related to the orientation of the first radial support structure 323. The first radial support structure 323 is basically parallel to the outer surface of the tube body 31 or turns outwardly relative to the outer surface of the tube body 31, so that the perpendicular distance from the edge of the proximal end (opening end) of the first radial support structure 323 to the outer surface of the tube body 31 is generally selected as the maximum perpendicular distance which is 6 to 40 mm, for example 6 to 30 mm.

The maximum length of the first radial support structure 323 is less than or equal to the maximum perpendicular distance from the first radial support structure 323 to the outer surface of the tube body 31, so that the flexible connecting section 322 in the radially compressed state easily actuates the opening end (namely the first radial support structure 323) of the stent graft 321 to bend relative to the flexible connecting section 322 towards a direction away from the axial direction 1b, thereby increasing the automatic bending success rate of the stent graft 321 and also improving the possibility that the first radial support structure 323 is perpendicular to the axial direction 1b of the tube body 31. The maximum length of the first radial support structure 323 is set to be 2 to 38 mm to ensure that the first radial support structure 323 has sufficient length to be adhered to the inner wall of the main body lumen stent 2 and also to avoid the first radial support structure 323 overlapping with adjacent side hole that will affect the implantation of the other branch lumen stents. Accordingly, the maximum perpendicular distance from the first radial support structure 323 to the outer surface of the tube body 31 is 6 to 40 mm.

In addition, when the opening end of the stent graft 321 is driven to bend under the radial compression of the flexible connecting section 322, the flexible connecting section 322 is basically adhered to the outer surface of the tube body 31, namely basically adhered to the outer surface of the first section 311 of the tube body 31. At this moment, the length of the flexible connecting section 322 is set to be less than that of the first section 311, so that at least a portion of the first section 311 of the tube body 31 is exposed relative to the skirt 32 after the stent graft 321 bends. After implantation, the proximal-end opening end 31b extends into the lumen of the main body lumen stent 2, and the exposed portion is located in the lumen of the main body lumen stent 2, so as to ensure that the blood flow in the main body lumen stent 2 may enter the branch lumen stent 3 through the proximal-end opening end 31b, thereby establishing blood flow of a branch lumen and preventing the main body lumen stent from moving. Meanwhile, the value of the difference between the length of the flexible connecting section 322 and the length of the first section 311 of the tube body 31 is set to be not more than 20 mm, which ensures that blood flows smoothly into the branch lumen stent 3, and blood turbulence or eddy currents are not caused in the main lumen stent 2, thereby minimizing the risk of thrombosis.

An implantation process of the lumen stent system of the present disclosure will be described below by taking the re-establishment of the blood supply between the aortic arch 11 and the left subclavian artery 12 from the aortic arch 11 as an example. The method of delivering the branch lumen stent on fig. 6-10 is not part of the present invention. The lumen stent system of the present disclosure may also be applicable to other vessels. For example, the lumen stent system of the present disclosure may be adopted to reestablish the blood supply between an abdominal aorta and a renal artery from the abdominal aorta, and no more descriptions will be provided here.

Referring to Fig. 6, during the implantation of the lumen stent system of the present disclosure, the main body lumen stent 2 is first implanted into a main body lumen (for example the aortic arch 11) using any proper technique, and the side hole 23 of the main body lumen stent 2 is aligned with the opening of a branch lumen (for example the left subclavian artery 12) from the main body lumen. Then, a delivery sheath 40 pre-loaded with the branch lumen stent 3 is delivered into the lumen of the main body lumen stent 2 from the left subclavian artery 12 through the side hole 23 of the main body lumen stent 2, and at this moment, the branch lumen stent 3 is radially compressed within the sheath 40.

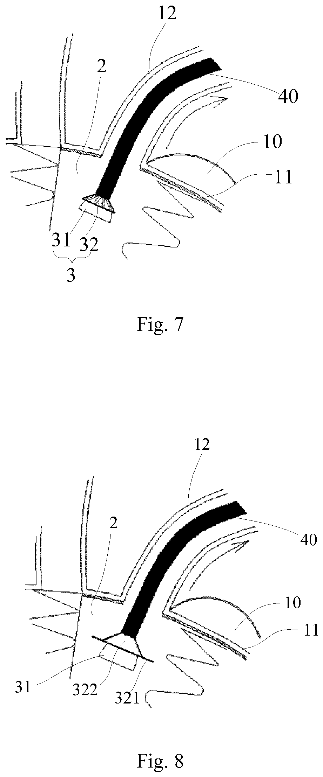

Referring to Fig. 7, the sheath 40 is withdrawn along the direction of the arrow to release the branch lumen stent 3, namely the branch lumen stent 3 is released step by step from its proximal end to distal end. For the skirt 32, the stent graft 321 is first released in the main body lumen stent 2. The stent graft 321 released from the sheath 40 is self-expanded to its initial shape and maintains its initial shape through the radial expansion capability of the first radial support structure 323. Similarly, the tube body 31 released in the main body lumen stent 2 is also self-expanded to its initial shape and maintains its initial shape through its radial support structure.

Referring to Fig. 8, the sheath 40 is continuously withdrawn until the stent graft 321 is completely released from the sheath 40, while at least a portion of the flexible connecting section 322 remains within the sheath 40. In other words, during the releasing process, after the stent graft 321 is completely released, the flexible connecting section 322 is still in a radially compressed state. Under the radial compression of the flexible connecting section 322, the released stent graft 321 bends relative to the flexible connecting section 322 and turns outwardly to enable the first radial support structure 323 of the stent graft 321 to be approximately perpendicular to the axial direction 1b of the tube body 31. As the length of the flexible connecting section 322 is greater than that of the first section 311 of the tube body 31, at least a portion of the tube body 31 is exposed relative to the skirt 32 after the stent graft 321 bends.

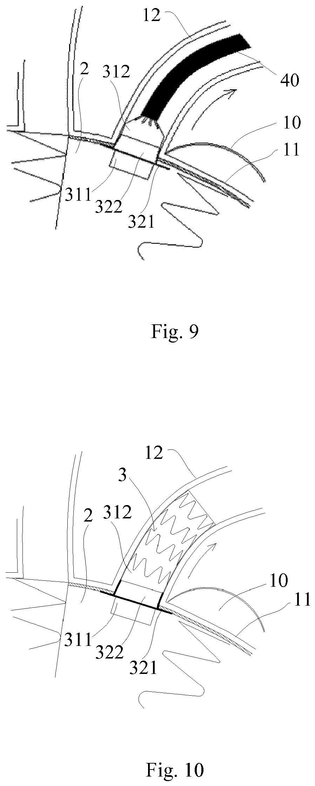

Referring to Fig. 9, after the stent graft 321 is completely released, the sheath 40 and the lumen stent system are moved together along the direction of the arrow during continuous withdrawal of the sheath 40 along the arrow in the Figure till the stent graft 321 is adhered to the inner side wall of the main body lumen stent 2, and then the sheath 40 may be pulled properly to enable the stent graft 321 to be more closely adhered to the inner side wall of the main body lumen stent 2.

Referring to Fig. 10, the branch lumen stent 3 is completely released from its proximal end to distal end, and the second section 312 and a portion of the first section 311 of the tube body 31 are implanted into the left subclavian artery 12. Furthermore, the branch lumen stent 3 may be stably located in the left subclavian artery 12 through the radial expansion capability of the tube body 31. The other portion of the second section 312 extends into the lumen of the main body lumen stent 2 through the side hole 23 of the main body lumen stent 2 to allow blood to enter the branch lumen stent 3. The flexible connecting section 322 of the skirt 32 and the tube body 31 are together radially compressed by the left subclavian artery 12. Under this radial compression, the stent graft 321 still bends relative to the flexible connecting section 322 and is closely adhered to the inner wall of the main body lumen stent 2 in the lumen of the main body lumen stent 2.

The stent graft 321 of the skirt 32 is equivalent to a brim of a traditional top hat stent, which may reduce the impact of the blood flow on their combined positions after the stent graft 321 is adhered to the inner wall of the main body lumen stent 2 such that the tube body 31 may maintain its radial support shape to avoid deformation such as wrinkling, introversion and collapse, thereby preventing the blood that flows into the lumen from being blocked to prevent formation of type-ill endoleak, and also reducing movement of the main body lumen stent 2 under the impact of the blood flow. Furthermore, on the side hole 23 of the main body lumen stent 2, a semi-closed gap is formed between the stent graft 321 of the skirt 32 and the tube body 31, and the blood flowing into the gap may be used as a filling material for occluding a type-III endoleak channel to prevent the blood from flowing into a aneurysm or a dissection 10. Moreover, the stent graft 321 used as the brim of the top hat stent that is separated from the tube body 31 is used as a blood flow inlet of the branch lumen stent 3, so that the blood flow inlet is not affected by conditions such as the shape of the side hole 23, whether the side hole 23 is concentric with the opening of the branch lumen or not, and the deformation or failure of the brim.

It should be noted that the branch lumen stent 3 may be also used independently in addition to cooperative use with the main body lumen stent 2. In other words, only the branch lumen stent 3 is implanted into the branch lumen (for example the left subclavian artery 12), namely the second section 312 and a portion of the first section 311 of its tube body 31 are implanted into the branch lumen, and the branch lumen stent 3 is stably located in the branch lumen through the radial expansion capacity of the tube body 31. The other portion of the second section 312 extends into the lumen of the main body lumen through the opening of the main body lumen (for example the aortic arch 11) to facilitate blood flow into the branch lumen stent 3. The flexible connecting section 322 of the skirt 32 and the tube body 31 are radially compressed by the branch lumen together. Under this radial compression, the opening end of the stent graft 321 still bends relative to the flexible connecting section 322 and is closely adhered to the inner wall of the main body lumen in the lumen of the main body lumen.

The following gives examples of comparative arrangements of branch stents, which are not part of the present invention.

### First example

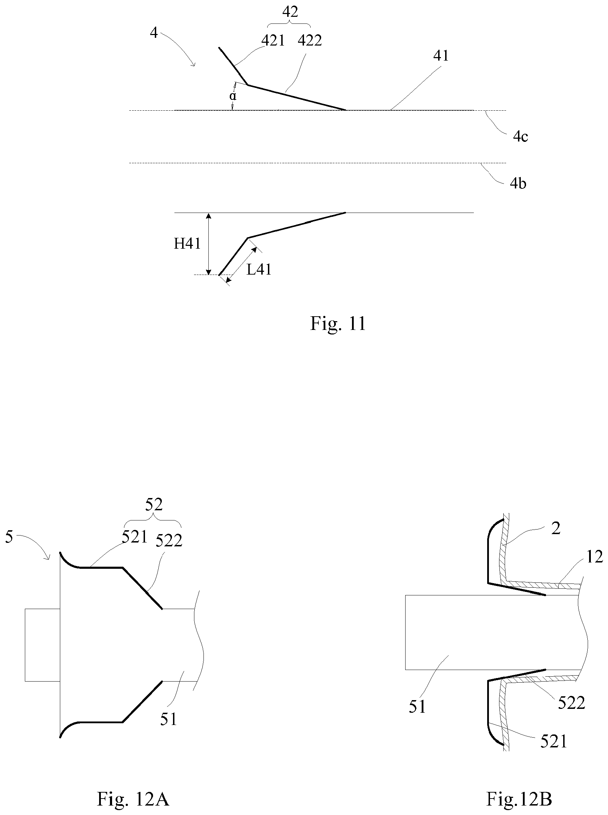

Referring to Fig. 11, a difference to the first embodiment is that according to a branch lumen stent 4 of the first example, along an opening direction of a skirt 42, namely along a direction from the distal end to the proximal end, an included angle between a stent graft 421 (namely a first radial support structure not shown in the figure) and an axial direction 4b of a tube body 41 is an acute angle, for example 60 degrees. Namely, in an initial state, the orientation of the stent graft 421 turns outwardly relative to the axial direction 1b of the tube body to form a horn shape. Furthermore, the included angle between the stent graft 421 and the axial direction 4b is greater than that between a flexible connecting section 422 and the axial direction 4b. The tube body 41 is the same as or similar to the tube body 31 in the first embodiment, so that no more details will be described.

By adopting this arrangement, the maximum perpendicular distance H41 from the stent graft 421 to the outer surface (namely the axial direction 4c of the outer surface) of the tube body 41 may be correspondingly increased. To this end, under the condition that the included angle α between the flexible connecting section 422 and the axial direction of the outer surface of the tube body 41 is relatively small, the maximum perpendicular distance H41 is also greater than the maximum length L41 of the first radial support structure. If the included angle α between the flexible connecting section 422 and the axial direction 4b is smaller, the branch lumen stent is released more successfully, and the force needed for pulling the sheath during release is smaller.

### Second example

Referring to Figs. 12A and 12B, a difference to the first embodiment is that the edge, namely the proximal end portion, of the suspended end (namely the first radial support structure that is not shown in the Figure) of a stent graft 521 of a skirt 52 of a branch lumen stent 5 according to the second example is everted in a natural state. A tube body 51 is the same as or similar to the tube body 31 in the first embodiment, so no more details will be described. After the branch lumen stent 5 is implanted, and when the stent graft 521 (the first radial support structure not shown in the figure) bends relative to a flexible connecting section 522, the outwardly turned edge may improve the adherence performance between the edge of the stent graft 521 and the inner wall of the main body lumen stent 2 so as to avoid formation of a leakage channel between the stent graft 521 and the inner wall of the main body lumen stent 2.

### Third example

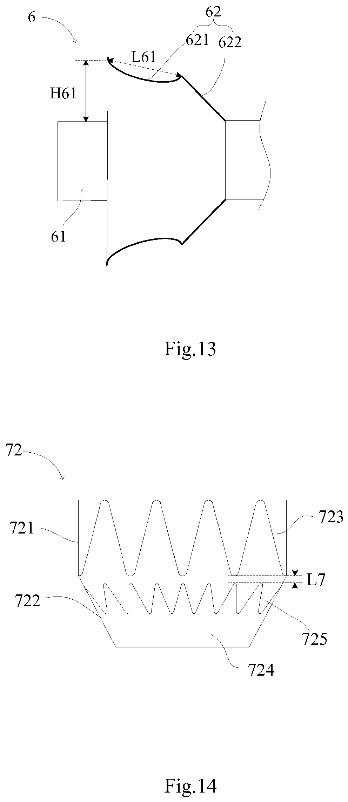

Referring to Fig. 13, a difference to the first embodiment is that a stent graft 621 (namely a first radial support structure that us not shown in the Figure) of a skirt 62 of a branch lumen stent 6 according to the third example is a concave curved surface, namely the diameter of the stent graft 621 is decreased progressively and then increased progressively according to a direction from a connecting boundary to the proximal end. After the branch lumen stent 6 is implanted, and when the stent graft 621 bends relative to a flexible connecting section 622, the concave curved surface may improve the adherence performance between the stent graft 621 and the inner wall of the main body lumen stent 2 so as to avoid formation of a leakage channel between the stent graft 621 and the inner wall of the main body lumen stent 2. The length L61 of the first radial support structure in the concave curved surface is equal to a length L61 of a connecting line between the proximal end portion and the distal end portion of the first radial support structure, and the maximum perpendicular distance from the first radial support structure to the outer surface of the tube body 61 is equal to a perpendicular distance H61 from the proximal end portion of the first radial support structure to the outer surface of the tube body 61, and still maintains the requirement that H61>L61. The tube body 61 is the same as or similar to the tube body 31 in the first embodiment, so no more details will be described.

### Fourth example

Referring to Fig. 14, a difference to the first embodiment is that a flexible connecting section 722 of a skirt 72 of a branch lumen stent according to the fourth example includes a second radial support structure 725 arranged along its circumferential direction. The second radial support structure 725 may be distributed on a portion of the flexible connecting section 722, namely the maximum length of the second radial support structure 725 is less than the length of the flexible connecting section 722 along the axial direction. The second radial support structure 725 also may be distributed over the entire flexible connecting section 722, namely the maximum length of the second radial support structure 725 is equal to the length of the flexible connecting section 722 along the axial direction. The second radial support structure 725 has a radial expansion capability, may be compressed under an external force, and restores to an initial shape through self-expansion and maintains the initial shape after the external force is withdrawn. The second radial support structure 725 may be made of various biocompatible materials including known materials used in manufacturing of the implantable medical device or a combination of various materials, such as 316L stainless steel, cobalt-chromium-nickel-molybdenum-iron alloy, other cobalt alloys such as L605, tantalum, nickel-titanium alloy (nitinol) or other biocompatible metals. The second radial support structure 725 may include a plurality of wave-shaped annuluses along the axial direction, such as Z-shaped waves, or include a helically wound structure, or include a mesh structure.

After the stent of the present example is implanted into a branch lumen, similarly, the flexible connecting section 722 and the tube body (not shown in the Figure) are radially compressed together by the branch lumen. The flexible connecting section 722 improves its adhesion to the wall of the branch lumen through the radial expansion capacity of the second radial support structure 725, thereby ensuring the smoothness of the gap between the flexible connecting section 722 and the tube body, so that blood can flow smoothly into the gap, and the sealing property is improved. Furthermore, the blood flow may be promoted to form a vortex under the action of pressure to change the direction, thereby facilitating the flow of the blood into the tube body.

The second radial support structure 725 may be formed by winding a metal wire having a diameter that less than that of a metal wire that is used to wind a first radial support structure 723, or less than a wire diameter of a metal rod formed by cutting to form the first radial support structure 723. Alternatively, the second radial support structure 725 may be formed by cutting a metal tube, and the wire diameter of the cut metal rod is less than the diameter of the metal wire that winds the first radial support structure 723, or less than that of the metal rod formed by cutting to form the first radial support structure 723. For example, if the second radial support structure 725 is formed by winding the metal wire, the diameter of the metal wire is 0.15 to 0.4 mm. Or, if the second radial support structure 725 is formed by cutting a metal tube, the wire diameter of the metal rod forming the second radial support structure 725 is 0.15 to 0.4 mm. The second radial support structure 725 has a relatively small wire diameter or rod diameter so as to reduce a friction force with the sheath, thereby reducing the release force of a delivery system. Furthermore, after implantation, the expansion force generated by the second radial support structure 725 also may be reduced. For example, the expansion force generated by the second radial support structure 725 on an opening of the branch lumen and/or main body lumen stent may be reduced.

In the present example, the second radial support structure 725 is distributed on a portion of the flexible connecting section 722, includes a second wave-shaped annulus which is a ring of Z-shaped waves, and is formed by winding a nickel-titanium alloy. The diameter of the metal wire is 0.1 mm. The first radial support structure 723 is basically distributed on the entire membrane-coated stent 721, includes a first wave-shaped annulus which is a ring of Z-shaped waves, and is formed by winding a nickel-titanium alloy. The diameter of the metal wire is 0.2 mm. Compared with the radial support force of other radial support structures, the radial support force of the Z-shaped waves is relatively high.

The waveform number of the first wave-shaped annulus is less than or equal to that of the second wave-shaped annulus. For example, the waveform number of the first wave-shaped annulus may be 5 to 12, and the waveform number of the second wave-shaped annulus may be twice that of the first wave-shaped annulus. Due to the arrangement of the waveform numbers, under the radial compression, the second wave-shaped annulus drives the first wave-shaped annulus more easily to bend and turn over towards a direction away from a first main body axial direction, and the adherence performance of the first wave-shaped annulus may be also guaranteed.

A coating membrane 724 of the skirt 72 covers both the first radial support structure 723 and the second radial support structure 725. The coating membrane 724 may be a PET membrane or a PTFE membrane, which may cover the first radial support structure 723 and the second radial support structure 725 after hot melting or suturing.

The shortest distance between the first radial support structure 723 and the second radial support structure 725 is less than or equal to 2 mm, namely the shortest distance between the adjacent first wave-shaped annulus and second wave-shaped annulus is less than or equal to 2mm. The shortest distance is a distance between a connecting line of all peaks of the first wave-shaped annulus and a connecting line of all valleys of the adjacent second wave-shaped annulus. In the present embodiment, referring to Fig, 14, the shortest length of the coating membrane length is equal to the shortest coating membrane gap L7 between one valley of the first wave-shaped annulus and the peak of the closest second wave-shaped annulus. Therefore, when radially compressed, the second radial support structure 725 may effectively assist the first radial support structure 723 to bend. If the shortest length of the coating membrane length between the first radial support structure 723 and the second radial support structure 725 is too long, it is difficult to transmit a radial compression force from the second radial support structure 725 to the first radial support structure 723, which is unfavorable for driving the first radial support structure 723 to bend.

### Fifth example

A difference to the fourth example is that a coating membrane or no coating membrane is arranged between a first radial support structure and a second radial support structure of a branch lumen stent according to the sixth embodiment. The bendable connection between a stent graft and a flexible connecting section is implemented through the bendable connection between the first radial support structure and the second radial support structure.

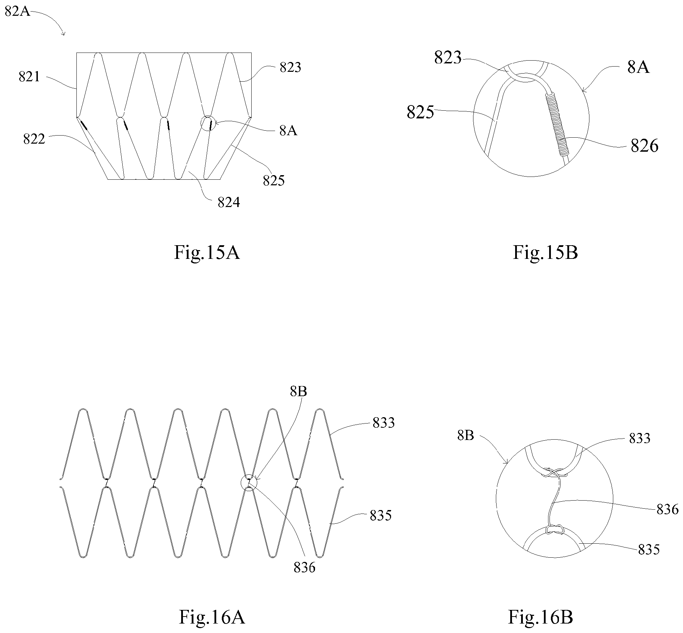

Referring to Figs. 15A and 15B, in one specific implementation mode of the fifth example, the first radial support structure 823 and the second radial support structure 825 are hooked and wound together, or are hung together. No coating membrane is arranged between the first radial support structure 823 and the second radial support structure 825, and the coating membrane 824 only covers a portion of the second radial support structure 825 and is sealed and connected with a tube body. Under the radial compression, the second radial support structure 825 may directly transmit a force to the first radial support structure 823, so that the first radial support structure 823 bends and turns over relative to the second radial support structure 825 more easily. To facilitate the implantation, a radiopaque apparatus may be arranged on the second radial support structure 825 to observe whether or not the first radial support structure 823 bends and turns over. The first radial support structure 823 includes at least a first wave-shaped annulus which may be a ring of Z-shaped waves. The second radial support structure 825 includes at least a second wave-shaped annulus which may be a ring of Z-shaped waves. Referring to an enlarged region 8A, the first wave-shaped annulus and the adjacent second wave-shaped annulus are hooked and wound together, namely the peak of one wave-shaped annulus is hung with the valley of the other wave-shaped annulus. Similarly, the waveform number of the first wave-shaped annulus is less than or equal to that of the second wave-shaped annulus. For example, the waveform number of the first wave-shaped annulus is equal to that of the second wave-shaped annulus in the Figure.

Referring to Figs. 16A and 16B, in another specific implementation mode of the sixth embodiment, a first radial support structure 833 and a second radial support structure 835 are connected through a flexible piece 836, and no coating membrane is arranged between them. The flexible piece 836 includes a biocompatible metal wire and/or macromolecular wire. For example, the metal wire may be a nickel-titanium alloy wire, and the macromolecular wire may be a PET suture or an ePTFE suture or other proper medical grade sutures. The flexible piece 836 may include a silk thread, or various silk threads used cooperatively. Referring to an enlarged region 8B, one end of the flexible piece is fixedly connected with the first radial support structure 833, and the other end of the flexible piece is connected with the second radial support structure 835. Similarly, the shortest distance between the first radial support structure 833 and the second radial support structure 835 along a skirt is less than or equal to 2mm. Namely, a distance between one valley of the first wave-shaped annulus and one peak of the closest second wave-shaped annulus is less than or equal to 2mm. Or, the length of the flexible piece also may be set to be less than or equal to 2 mm. Therefore, when radially compressed, the second radial support structure 835 may effectively assist the first radial support structure 833 to bend.

In conclusion, the stent graft of the skirt of the branch lumen stentaccording to the present invention includes a first radial support structure, and the first radial support structure is bendably connected to the flexible connecting section, so that during implantation and after implantation, under radial compression of the flexible connecting section, the first radial support structure bends relative to the flexible connecting section. The radial support structure bends to become approximately perpendicular to the axial direction of the tube body in the implanted condition. The first radial support structure acts as the brim of the traditional top hat stent, which may be adhered to the inner wall of the main body lumen stent to reduce the impact of the blood flow to their combined positions so as to enable the tube body to maintain its radial support shape and avoid the deformation such as wrinkling, introversion and collapse, thereby avoiding resistance to the blood flowing into the lumen and preventing the formation of type-III endoleak. Meanwhile, the brim also may reduce the movement of the main body lumen stent under the impact of the blood flow.

Further, on the side hole of the main body lumen stent, a semi-closed gap is formed between the stent graft of the skirt and the tube body, and blood which flows into the gap may be used as the filling material for occluding the type-III endoleak channel to prevent the formation of the leakage channels between the tube body and the wall of the branch lumen as well as between the tube body and the opening of the branch lumen, thereby preventing the blood from flowing into the aneurysm or the dissection.

Further, the stent graft used as the brim of the top hat stent is separated from the tube body 31 used as the blood flow inlet of the branch lumen stent, so that the function of the blood flow inlet is not affected by the shape of the side hole of the main body lumen stent, whether the side hole is concentric with the opening of the branch lumen or not, and the deformation or failure of the brim. This ensures smooth blood flow into the branch lumen stent.

## Claims

1. A branch lumen stent (3) suitable for implantation into a main body lumen stent (2), comprising a tube body (31) and a skirt (32) arranged on the tube body (31) with the skirt (32) surrounding the tube body (31), the tube body (31) having an outer surface, wherein the skirt (32) comprises a flexible connecting section (322) and a stent graft (321), the flexible connecting section (322) having a distal end and a proximal end, and the stent graft (321) having a proximal end,
wherein the proximal end of the flexible connecting section (322) is connected with the stent graft (321), and the distal end of the flexible connecting section (322) is sealed and connected with the outer surface of the tube body (31), and
wherein the proximal end of the stent graft (321) is suspended and provided with a first radial support structure (323), wherein
when implanted, the flexible connecting section (322) actuates the stent graft (321) to bend under radial compression of the branch lumen so that the first radial support structure (323) is approximately perpendicular relative to the axial direction (1b) of the tube body (31), and can be adhered to the inner tube wall of the main body lumen stent (2).

2. The branch lumen stent according to claim 1, wherein an included angle is defined between the flexible connecting section (322) and the axial direction (1c) of the outer surface of the tube body (31), and the included angle is 5 to 80 degrees.

3. The branch lumen stent according to claim 1, wherein the first radial support structure (323) has a maximum axial length (L21), and a maximum perpendicular distance (H21) is defined from the edge of the first radial support structure (323) to the outer surface of the tube body (31), wherein the maximum axial length of the first radial support structure (323) is less than or equal to the maximum perpendicular distance from the edge of the first radial support structure (323) to the outer surface of the tube body (31).

4. The branch lumen stent according to claim 3, wherein the maximum perpendicular distance (H21) from the edge of the first radial support structure (323) to the outer surface of the tube body (31) is 6 to 40 mm, and the maximum axial length (L21) of the first radial support structure (323) is 2 to 38 mm.

5. The branch lumen stent according to claim 1, wherein the flexible connecting section (322) has an axial length and a connecting boundary (31a) that connects with the tube body (31), and the tube body (31) has a proximal end surface and a length (L11) defined between the proximal end surface of the tube body (31) and the connecting boundary (31a), wherein the axial length of the flexible connecting section (322) is less than the length between the proximal end surface of the tube body (31) and the connecting boundary.

6. The branch lumen stent according to claim 5, wherein the difference between the length from the proximal end surface of the tube body (31) to the connecting boundary (31a) and the axial length of the flexible connecting section (322) is not more than 20 mm.

7. The branch lumen stent according to claim 1, wherein at least a portion of the first radial support structure (323) is covered by a coating membrane (324).

8. The branch lumen stent according to claim 1, wherein the flexible connecting section (322) comprises a coating membrane (324) that has two ends, wherein one end of the coating membrane is sealed and connected with the outer surface of the tube body (31), and the other end of the coating membrane is connected with the stent graft (321).

9. A lumen stent system, comprising the branch lumen stent (3) according to any one of claims 1 to 8 and a main body lumen stent (2), wherein the main body lumen stent has an inner wall and is provided with a side hole (23), wherein the tube body (31) of the branch lumen stent (3) passes through the side hole (23) and the flexible connecting section (322) of the branch lumen stent (3) is radially compressed by the branch lumen, so that the first radial support structure (323) is approximately perpendicular to the axial direction of the tube body (31) and can be adhered to the inner wall of the main body lumen stent (2).

## Patentansprüche

1. Zweiglumenstent (3), der zur Implantation in einen Hauptkörperlumenstent (2) geeignet ist, mit einem Schlauchkörper (31) und einer an dem Schlauchkörper (31) angeordneten Schürze (32), wobei die Schürze (32) den Schlauchkörper (31) umgibt, wobei der Schlauchkörper (31) eine Außenfläche aufweist, wobei die Schürze (32) einen flexiblen Verbindungsabschnitt (322) und ein Stenttransplantat (321) umfasst, wobei der flexible Verbindungsabschnitt (322) ein distales Ende und ein proximales Ende aufweist und das Stenttransplantat (321) ein proximales Ende aufweist,
wobei das proximale Ende des flexiblen Verbindungsabschnitts (322) mit dem Stenttransplantat (321) verbunden ist und das distale Ende des flexiblen Verbindungsabschnitts (322) verschlossen und mit der Außenfläche des Schlauchkörpers (31) verbunden ist, und
wobei das proximale Ende des Stenttransplantats (321) hängend und mit einer ersten radialen Tragstruktur (323) versehen ist, wobei
der flexible Verbindungsabschnitt (322) dann, wenn er implantiert ist, das Stenttransplantat (321) dazu veranlasst, sich unter radialer Kompression des Zweiglumens so zu biegen, dass die erste radiale Tragstruktur (323) annähernd senkrecht zur Axialrichtung (1b) des Schlauchkörpers (31) ist und mit der Innenschlauchwand des Hauptkörperlumenstents (2) verklebt werden kann.

2. Zweiglumenstent nach Anspruch 1, bei dem zwischen dem flexiblen Verbindungsabschnitt (322) und der Axialrichtung (1c) der Außenfläche des Schlauchkörpers (31) ein eingeschlossener Winkel definiert ist und der eingeschlossene Winkel 5 bis 80 Grad beträgt.

3. Zweiglumenstent nach Anspruch 1, bei dem die erste radiale Tragstruktur (323) eine maximale axiale Länge (L21) aufweist und ein maximaler senkrechter Abstand (H21) vom Rand der ersten radialen Tragstruktur (323) zur Außenfläche des Schlauchkörpers (31) definiert ist, wobei die maximale axiale Länge der ersten radialen Tragstruktur (323) kleiner oder gleich dem maximalen senkrechten Abstand vom Rand der ersten radialen Tragstruktur (323) zur Außenfläche des Schlauchkörpers (31) ist.

4. Zweiglumenstent nach Anspruch 3, bei dem der maximale senkrechte Abstand (H21) vom Rand der ersten radialen Tragstruktur (323) zur Außenfläche des Schlauchkörpers (31) 6 bis 40 mm beträgt und die maximale axiale Länge (L21) der ersten radialen Tragstruktur (323) 2 bis 38 mm beträgt.

5. Zweiglumenstent nach Anspruch 1, bei dem der flexible Verbindungsabschnitt (322) eine axiale Länge und eine Verbindungsbegrenzung (31a) aufweist, die an den Schlauchkörper (31) angeschlossen ist, und der Schlauchkörper (31) eine proximale Endfläche und eine Länge (L11) aufweist, die zwischen der proximalen Endfläche des Schlauchkörpers (31) und der Verbindungsbegrenzung (31a) definiert ist, wobei die axiale Länge des flexiblen Verbindungsabschnitts (322) kleiner als die Länge zwischen der proximalen Endfläche des Schlauchkörpers (31) und der Verbindungsbegrenzung ist.

6. Zweiglumenstent nach Anspruch 5, bei dem die Differenz zwischen der Länge von der proximalen Endfläche des Schlauchkörpers (31) zur Verbindungsbegrenzung (31a) und der axialen Länge des flexiblen Verbindungsabschnitts (322) nicht mehr als 20 mm beträgt.

7. Zweiglumenstent nach Anspruch 1, bei dem wenigstens ein Teil der ersten radialen Tragstruktur (323) von einer Beschichtungsmembran (324) bedeckt ist.

8. Zweiglumenstent nach Anspruch 1, bei dem der flexible Verbindungsabschnitt (322) eine Beschichtungsmembran (324) umfasst, die zwei Enden aufweist, wobei ein Ende der Beschichtungsmembran verschlossen und mit der Außenfläche des Schlauchkörpers (31) verbunden ist und das andere Ende der Beschichtungsmembran mit dem Stenttransplantat (321) verbunden ist.

9. Lumenstentsystem, mit dem Zweiglumenstent (3) nach einem der Ansprüche 1 bis 8 und einem Hauptkörperlumenstent (2), wobei der Hauptkörperlumenstent eine Innenwand aufweist und mit einem seitlichen Loch (23) versehen ist, wobei der Schlauchkörper (31) des Zweiglumenstents (3) das seitliche Loch (23) durchtritt und der flexible Verbindungsabschnitt (322) des Zweiglumenstents (3) von dem Zweiglumen radial zusammengedrückt ist, so dass die erste radiale Tragstruktur (323) annähernd senkrecht zur Axialrichtung des Schlauchkörpers (31) ist und mit der Innenwand des Hauptkörperlumenstents (2) verklebt werden kann.

## Revendications

1. Stent de lumière de branchement (3) adapté à l'implantation dans un stent de lumière de corps principal (2), comprenant un corps tubulaire (31) et une jupe (32) agencée sur le corps tubulaire (31), la jupe (32) entourant le corps tubulaire (31), le corps tubulaire (31) présentant une surface extérieure, la jupe (32) comprenant une partie de raccordement flexible (322) et un greffon de stent (321), la partie de raccordement flexible (322) présentant une extrémité distale et une extrémité proximale, et le greffon de stent (321) présentant une extrémité proximale,
l'extrémité proximale de la partie de raccordement flexible (322) étant raccordée au greffon de stent (321) et l'extrémité distale de la partie de raccordement flexible (322) étant fermée et raccordée à la surface extérieure du corps tubulaire (31), et
l'extrémité proximale du greffon de stent (321) étant suspendue et pourvue d'une première structure de support radiale (323),
la partie de raccordement flexible (322), lorsqu'elle est implantée, amenant le greffon de stent (321) à se courber sous la compression radiale de la lumière de branchement de sorte que la première structure de support radiale (323) est approximativement perpendiculaire au sens axial (1b) du corps tubulaire (31) et peut être collée à la paroi tubulaire interne du stent de lumière de corps principal (2).

2. Stent de lumière de branchement selon la revendication 1, un angle inclus étant défini entre la partie de raccordement flexible (322) et le sens axial (1c) de la surface extérieure du corps tubulaire (31), et l'angle inclus étant de 5 à 80 degrés.

3. Stent de lumière de branchement selon la revendication 1, la première structure de support radiale (323) présentant une longueur axiale maximale (L21), et une distance perpendiculaire maximale (H21) étant définie depuis le bord de la première structure de support radiale (323) jusqu'à la surface extérieure du corps tubulaire (31), la longueur axiale maximale de la première structure de support radiale (323) étant inférieure ou égale à la distance perpendiculaire maximale depuis le bord de la première structure de support radiale (323) jusqu'à la surface extérieure du corps tubulaire (31).

4. Stent de lumière de branchement selon la revendication 3, la distance perpendiculaire maximale (H21) depuis le bord de la première structure de support radiale (323) à la surface extérieure du corps tubulaire (31) étant de 6 à 40 mm et la longueur axiale maximale (L21) de la première structure de support radiale (323) étant de 2 à 38 mm.

5. Stent de lumière de branchement selon la revendication 1, la partie de raccordement flexible (322) présentant une longueur axiale et une limite de raccordement (31a) raccordée au corps tubulaire (31), et le corps tubulaire (31) présentant une surface d'extrémité proximale et une longueur (L11) définie entre la surface d'extrémité proximale du corps tubulaire (31) et la limite de raccordement (31a), la longueur axiale de la partie de raccordement flexible (322) étant inférieure à la longueur entre la surface d'extrémité proximale du corps tubulaire (31) et la limite de raccordement.

6. Stent de lumière de branchement selon la revendication 5, la différence entre la longueur de la surface d'extrémité proximale du corps tubulaire (31) à la limite de raccordement (31a) et la longueur axiale de la partie de raccordement flexible (322) n'étant pas supérieure à 20 mm.

7. Stent de lumière de branchement selon la revendication 1, au moins une partie de la première structure de support radiale (323) étant recouverte d'une membrane de revêtement (324).

8. Stent de lumière de branchement selon la revendication 1, la partie de raccordement flexible (322) comprenant une membrane de revêtement (324) qui présente deux extrémités, une extrémité de la membrane de revêtement étant fermée et raccordée à la surface extérieure du corps tubulaire (31) et l'autre extrémité de la membrane de revêtement étant raccordée au greffon de stent (321).

9. Système de stent de lumière, comprenant le stent de lumière de branchement (3) selon l'une des revendications 1 à 8 et un stent de lumière de corps principal (2), le stent de lumière de corps principal comprenant une paroi interne et étant pourvue d'un trou latéral (23), le corps tubulaire (31) du stent de lumière de branchement (3) traversant le trou latéral (23), et la partie de raccordement flexible (322) du stent de lumière de branchement (3) étant comprimée radialement par la lumière de branchement, de sorte que la première structure de support radiale (323) est approximativement perpendiculaire au sens axial du corps tubulaire (31) et peut être collée à la paroi interne du stent de lumière de corps principal (2).
